(19) Europäisches Patentamt
European Patent Office
Office européen des brevets

(11) EP 2 189 151 B1

(12) # FASCICULE DE BREVET EUROPEEN

(45) Date de publication et mention de la délivrance du brevet:
**22.11.2017 Bulletin 2017/47**

(51) Int Cl.:
***A61K 8/81*** *(2006.01)* ***A61Q 1/02*** *(2006.01)*
***A61Q 1/06*** *(2006.01)* ***A61Q 1/12*** *(2006.01)*

(21) Numéro de dépôt: **09173746.0**

(22) Date de dépôt: **22.10.2009**

(54) **Procédé de maquillage de la peau et/ou des lèvres employant une composition comprenant un polymère supramoléculaire**

Verfahren zum schminken und auftragen einer zusammensetzung enthaltend ein supramolekulares polymer

Process for the makeup of skin and/or lips by applying a composition comprising a supramolecular polymer

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **24.11.2008 FR 0857939**

(43) Date de publication de la demande:
**26.05.2010 Bulletin 2010/21**

(73) Titulaire: **L'Oréal**
**75008 Paris (FR)**

(72) Inventeurs:
- **Chodorowski-Kimmes, Sandrine**
**60300, Senlis (FR)**
- **Arnaud, Pascal**
**94240, L'Hay Les Roses (FR)**
- **Barba, Claudia**
**75010, Paris (FR)**

(74) Mandataire: **Kromer, Christophe**
**L'Oréal**
**Service DIPI**
**9 Rue Pierre Dreyfus**
**92110 Clichy (FR)**

(56) Documents cités:
**EP-A- 1 797 868** **WO-A-03/032929**
**WO-A-2007/039832** **FR-A- 2 825 628**

Remarques:
Le dossier contient des informations techniques présentées postérieurement au dépôt de la demande et ne figurant pas dans le présent fascicule.

EP 2 189 151 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

## Description

La présente invention a pour objet une composition de maquillage de la peau, notamment du visage, ou bien des lèvres, comprenant un ou plusieurs polymères supramoléculaires à base de polyalcène. L'invention concerne également un procédé de maquillage de la peau et/ou des lèvres comprenant l'application d'une telle composition.

Lorsque les femmes utilisent un produit de teint, notamment de type fond de teint, elles souhaitent que ce produit présente, après application, une bonne tenue sur la peau, en particulier qu'il ne transfère pas sur les vêtements. Il est connu de l'homme de l'art d'utiliser des polymères pour obtenir ces propriétés de longue tenue au cours de la journée. Ces polymères sont de natures chimiques très différentes et sont généralement véhiculés soit dans une phase grasse, soit dans une phase aqueuse. On peut citer comme exemples, les résines de silicones, les polyacrylates et les latex. Si ces polymères apportent effectivement des propriétés de tenue, en particulier de non transfert, celles-ci s'accompagnent le plus souvent d'inconfort, par exemple lors de l'application du produit (étalement difficile, collant) et/ou au cours de la journée (tiraillements, effet masque).

Dans le domaine des rouges à lèvres, la bonne tenue de la composition est également une demande des consommatrices. Elle est généralement obtenue en utilisant des polymères dits filmogènes, de façon à limiter le transfert de couleur. La réalisation de films de polymère sur les lèvres peut toutefois être source d'inconfort en particulier du fait des tensions qu'exerce le polymère filmogène sur la muqueuse labiale.
Par ailleurs, les compositions de maquillage des lèvres sont pour la plupart brillantes ou présentent un effet brillant. Cette brillance qui permet de mettre les lèvres en valeur est généralement obtenue par la formulation d'huiles brillantes et/ou de particules à reflets. Toutefois, l'utilisation d'huiles brillantes peut générer du collant, ce caractère collant conduisant ces compositions à laisser des traces sur les supports comme les verres, les tasses à café, donc à transférer.

Il subsiste donc le besoin de disposer de polymères susceptibles d'être employés pour le maquillage du teint et permettant d'obtenir à la fois de bonnes propriétés de tenue tout en conservant un certain confort à l'usage.
Il subsiste aussi le besoin de disposer de polymères susceptibles d'être employés pour le maquillage des lèvres et permettant d'obtenir à la fois un dépôt confortable, brillant et non transfert.

La présente invention a pour but de proposer de tels polymères.

Un objet de l'invention est donc un procédé de maquillage de la peau et/ou des lèvres, dans lequel on applique sur la peau et/ou les lèvres une composition cosmétique de maquillage comprenant, dans un milieu cosmétiquement acceptable, métique de maquillage comprenant, dans un milieu cosmétiquement acceptable, au moins un polymère supramoléculaire à base de polyalcène selon la revendication 1.
Un autre objet de l'invention est une composition cosmétique de maquillage de la peau et/ou des lèvres, comprenant, dans un milieu cosmétiquement acceptable, au moins un polymère supramoléculaire à base de polyalcène, et au moins une matière colorante.

Les polymères dits "supramoléculaires" sont connus d'une manière générale en cosmétique.
On peut notamment citer WO02/09837 qui décrit de nombreux polymères aptes à former après application sur un substrat kératinique des polymères supramoléculaires. Les exemples ne décrivent pas spécifiquement de produits de maquillage pour la peau et/ou les lèvres, présentant une bonne tenue; par ailleurs, les polymères de l'invention y sont mentionnés de manière générale, et sans aucune concentration donnée pour le polymère.
Le document WO03/032929 décrit des compositions cosmétiques capillaires, comprenant des polymères supramoléculaires. Il n'y est pas mentionné d'utilisation dans le domaine du maquillage, notamment pour obtenir une bonne tenue et un certain confort.
Le document P1797868 décrit des compositions cosmétiques, notamment de maquillage, comprenant un copolymère (thio)uréthane/(thio)urée capable de former au moins trois liaisons hydrogène; toutefois, il n'est pas mentionné que ces polymères permettent d'obtenir une composition de bonne tenue ou confortable à porter.

On constate donc que rien dans l'art antérieur ne suggère d'utiliser des polymères tels que ceux définis dans la présente invention, pour obtenir une composition de maquillage pour la peau ou les lèvres présentant des propriétés de non transfert et de tenue adéquates, sans entrainer de problèmes d'inconfort. L'apport de brillance grâce aux polymères de l'invention n'est pas non plus suggérée dans l'art antérieur.

Les compositions cosmétiques selon l'invention comprennent donc un polymère supramoléculaire à base de polyalcène selon la revendication 1.
Au sens de la présente invention, on entend par polymère supramoléculaire à base de polyalcène, un polymère issu de la réaction, notamment de la condensation, d'au moins un polymère polyalcène fonctionnalisé par au moins un groupe réactif, avec au moins un groupe de jonction fonctionnalisé par au moins un groupe réactif susceptible de réagir avec le ou les groupes réactifs du polymère polyalcène fonctionnalisé, ledit groupe de jonction étant capable de former au moins 3 liaisons H (hydrogène), de préférence au moins 4 liaisons H, préférentiellement 4 liaisons H.

Le polymère supramoléculaire selon l'invention est susceptible de former une chaîne ou un réseau polymérique supramoléculaire, par (auto)assemblage dudit polymère selon l'invention avec au moins un autre polymère selon l'invention, identique ou différent, chaque assemblage faisant intervenir au moins une paire de groupes de jonction appariés,

identiques ou différents, portés par chacun des polymères selon l'invention.
Par groupe de jonction, on entend au sens de l'invention, tout groupe comportant des groupes donneurs ou accepteurs de liaisons H, et capable d'établir au moins trois liaisons H, de préférence au moins 4 liaisons H, préférentiellement 4 liaisons H, avec un groupe de jonction partenaire, identique ou non. Ces groupes de jonction peuvent être latéraux au squelette polymérique (en ramification latérale), et/ou portés par les extrémités du squelette polymérique, et/ou dans la chaîne formant le squelette polymérique. Ils peuvent être répartis de façon aléatoire ou contrôlée,
le polymère polyalcène fonctionnalisé, susceptible de former tout ou partie du squelette polymérique du polymère supramoléculaire selon l'invention, est de formule HX-P-X'H dans laquelle :

- XH et X'H sont des groupes réactifs, avec X et X' désignent O ;
- P représente un polyéthylène, un polybutylène, un polybutadiène, un polyisoprène, un poly(1,3-pentadiène), un polyisobutylène, et leurs copolymères, et notamment un poly(éthylènebutylène).

Les poly(éthylènebutylène) sont des copolymères de butène-1 et d'éthylène. On peut les schématiser par l'enchaînement de motifs suivants :

[-$CH_2$-$CH_2$-] et [-$CH_2CH(CH_2$-$CH_3)$-]

Les polybutadiènes peuvent être des 1,4-polybutadiènes ou des 1,2-polybutadiènes, qui peuvent être respectivement schématisés par les enchaînements de motifs suivants :

[-$CH_2$-CH=CH-$CH_2$-] (polybutadiènes 1,4)
[-$CH_2$-CH(CH=$CH_2$)-] (polybutadiènes 1,2)

De préférence, il s'agit de polybutadiènes 1,2.
Les polyisoprènes peuvent être schématisés par les enchainements de motifs suivants :

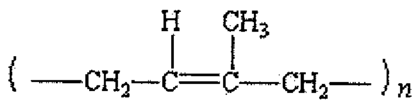
et
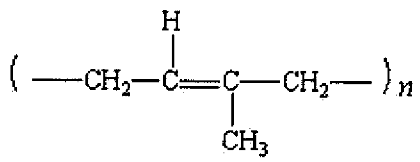

On peut bien évidemment également utiliser un mélange de motifs ci-dessus, afin de former des copolymères.
De préférence, les polymères polyalcènes fonctionnalisés présentent une massue moléculaire en nombre (Mn) supérieure ou égaie à 1000, notamment comprise entre 1000 et 5000, voire entre 1500 et 3500.
Les polymères supramoléculaires selon l'invention possèdent également dans leur structure au moins un reste d'un groupe de jonction capable de former au moins 3 liaisons H, de préférence au moins 4 liaisons H, ledit groupe de jonction étant initialement fonctionnalisé par au moins un groupe réactif.
Sauf précision contraire, on entend dans la présente description par groupe de jonction, le groupe sans sa fonction réactive.
Les groupes de jonction fonctionnalisés, susceptibles de réagir avec le polymère polyalcène fonctionnalisé pour donner le polymère supramoléculaire selon l'invention, sont de formule :

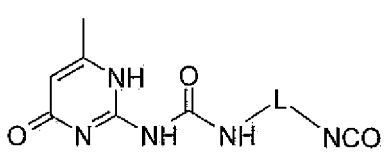

dans laquelle L est un groupe divalent isophorone ou 4,4'-méthylène biscyclohexylène.
le polymère supramoléculaire de l'invention répond à la formule :

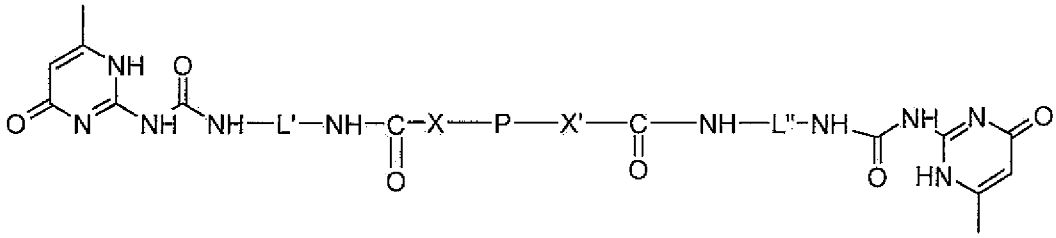

dans laquelle :

- L' et L" sont,un groupement -isophorone- ou L et L' sont un groupement 4,4'-méthylène biscyclohexylène ; et
- X et X' désignent O ; et
- P représente un polyéthylène, un polybutylène, un polybutadiène, un polyisoprène, un poly(1,3-pentadiène), un polyisobutylène, et leurs copolymères.

Le polymère selon l'invention peut être préparé par les procédés usuellement employés par l'homme du métier, notamment pour former une liaison uréthanne entre les fonctions OH libres d'un polyalcène, et les fonctions isocyanates portées par le groupe de jonction.
A titre d'illustration, un premier procédé général de préparation consiste à :

- éventuellement s'assurer que le polymère à fonctionnaliser ne comporte pas d'eau résiduelle,
- chauffer ledit polymère comportant au moins une fonction réactive, notamment 2 fonctions réactives, en particulier OH, à une température pouvant être comprise entre 60°C et 140°C; l'indice d'hydroxyle du polymère pouvant servir de référence afin de mesurer l'état d'avancement de la réaction ;
- ajouter, de préférence directement, le groupe de jonction portant les fonctions réactives, notamment isocyanate;
- éventuellement agiter le mélange, sous atmosphère contrôlée, à une température de l'ordre de 90-130°C; pendant 1 à 24 heures;
- éventuellement suivre par spectroscopie infrarouge, la disparition de la bande caractéristique des isocyanates (compris entre 2500 et 2800 cm-1) de manière à arrêter la réaction à la disparition totale du pic, puis à laisser revenir à température ambiante le produit final.

La réaction peut également être suivie par dosages des fonctions hydroxyles ; il est aussi possible d'ajouter de l'éthanol afin de s'assurer de la disparition totale des fonctions isocyanate résiduelles.
La réaction peut être effectuée en présence d'un solvant, notamment la méthyltétrahydrofurane, le tétrahydrofurane, le toluène, le propylène carbonate ou l'acétate de butyle. Il est également possible d'ajouter un catalyseur conventionnel de la formation de liaison uréthane. A titre d'exemple, on peut citer le dilaurate dibutyle étain. Le polymère peut au final être lavé et séché, voire purifié, selon les connaissances générales de l'homme du métier.
Selon le 2$^{ème}$ mode de préparation, la réaction peut comporter les étapes suivantes:

(i) fonctionnalisation du polymère de préférence préalablement séché, par un diisocyanate selon le schéma réactionnel :

OH-polymère-OH (1 éq.) + NCO-X-NCO (1 éq.) $\rightarrow$ OCN-X-NH-(O)CO-polymère-OC(O)-NH-X-NCO

Le diisocyanate peut être éventuellement en excès par rapport au polymère. Cette première étape peut être faite en présence de solvant, à une température comprise entre 20°C et 100°C. Cette première étape peut être suivie par une période d'agitation, sous atmosphère contrôlée pendant 1 à 24 heures. Le mélange peut être éventuellement chauffé. L'état d'avancement de cette première étape peut être suivi par un dosage des fonctions hydroxyles.
puis
(ii) réaction du prépolymère obtenu ci-dessus avec la 6-méthylisocytosine de formule :

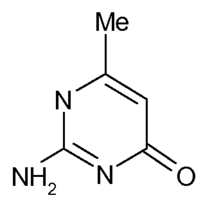

cette deuxième étape peut éventuellement se faire en présence d'un co-solvant comme le toluène, l'acétate de butyle ou le propylène carbonate. Le mélange réactionnel peut être chauffé entre 80°C et 140°C pendant un temps variant entre 1 à 24 heures. La présence d'un catalyseur, notamment le dilaurate dibutyle étain, peut favoriser l'obtention du produit final désiré.

La réaction peut être suivie par spectroscopie infrarouge, en suivant la disparition du pic caractéristique de l'isocyanate entre 2200 et 2300 cm-1. A la fin de la réaction, on peut ajouter de l'éthanol au milieu réactionnel afin de neutraliser les éventuelles fonctions isocyanate résiduelles. Le mélange réactionnel peut être éventuellement filtré. Le polymère peut également directement être strippé dans un solvant cosmétique.

Le polymère supramoléculaire à base de polyalcène, seul ou en mélange, peut être présent dans la composition cosmétique de maquillage selon l'invention à raison de 0,1 à 70% en poids, de préférence de 0,2 à 40% en poids, préférentiellement de 0,5 à 15% en poids, par rapport au poids total de la composition.

Dans un mode de réalisation particulier de l'invention, la composition de maquillage se présente sous forme d'un fond de teint et le polymère supramoléculaire à base de polyalcène, seul ou en mélange, peut y être présent à raison de 0,1 à 10% en poids, de préférence de 0,2 à 5% en poids, préférentiellement de 0,5 à 3% en poids, par rapport au poids total de la composition.

Dans un autre mode de réalisation particulier de l'invention, la composition de maquillage se présente sous forme d'un rouge à lèvres et le polymère supramoléculaire à base de polyalcène, seul ou en mélange, peut y être présent à raison de 0,1 à 70% en poids, de préférence de 0,2 à 40% en poids, préférentiellement de 0,5 à 15% en poids, par rapport au poids total de la composition.

La composition cosmétique de maquillage selon l'invention comprend en outre un milieu cosmétiquement acceptable qui peut comprendre les ingrédients usuels, en fonction de la destination de la composition.

De préférence, la composition de maquillage de la peau et/ou des lèvres selon l'invention, comprend au moins une matière colorante, qui peut être choisie parmi les pigments et les colorants, hydrosolubles ou liposolubles.

Par pigment, on entend tous les pigments apportant de la couleur aux matières kératiniques. Leur solubilité dans l'eau à 25°C et à pression atmosphérique (760 mm Hg) est inférieure à 0,05 % en poids, et de préférence inférieure à 0,01%.

Les pigments qui peuvent être utilisés sont notamment choisis parmi les pigments organiques ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome, le bleu ferrique et l'oxyde de titane.

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante

de dérivés indoliques, phénoliques tels qu'ils sont décrits dans le brevet FR 2 679 771.
Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.
Les substrats inorganiques sur lesquels sont adsorbés les colorants sont par exemple l'alumine, la silice, le borosilicate de calcium et de sodium ou le borosilicate de calcium et d'aluminium, et l'aluminium.
Parmi les colorants, on peut citer le carmin de cochenille. On peut également citer les colorants connus sous les dénominations suivantes: D&C Red 21 (CI 45380), D&C Orange 5 (CI 45370), D&C Red 27 (CI 45410), D&C Orange 10 (CI 45425), D&C Red 3 (CI 45430), D&C Red 4 (CI 15510), D&C Red 33 (CI 17200), FD&C Yellow 5 (CI 19140), FD&C Yellow 6 (CI 15985), D&C Green 5 (CI 61570), D&C Yellow 10 (CI 77002), FD&C Green 3 (CI 42053), FD&C Blue 1 (CI 42090). A titre d'exemples de laques, on peut citer les laques de calcium de D&C Red 7, 11, 31 ou 34; la laque de baryum de D&C Red 12; la laque de strontium D&C Red 13; les laques d'aluminium de FD&C Yellow 5, de FD&C Yellow 6, de D&C Red 27, de D&C Red 21, et de FD&C Blue 1.

Le pigment peut aussi être un pigment à effets spéciaux. Par pigment à effets spéciaux, on entend un pigment qui crée d'une manière générale une apparence colorée (caractérisée par une certaine nuance, une certaine vivacité et une certaine clarté) non uniforme et changeante en fonction des conditions d'observation (lumière, température, angles d'observation notamment). Il s'oppose par-là même aux pigments colorés qui procurent une teinte uniforme opaque, semi-transparente ou transparente classique.
Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.
A titre d'exemples de pigments à effets spéciaux, on peut citer les pigments nacrés tels que le mica-titane recouvert d'oxyde de fer, le mica recouvert d'oxyde de fer, le mica recouvert d'oxychlorure de bismuth, le mica titane recouvert d'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.
Les nacres peuvent plus particulièrement posséder une couleur ou un reflet jaune, rose, rouge, bronze, orangé, brun, or et/ou cuivré.
Parmi les nacres, on peut notamment citer les nacres de couleur or notamment commercialisées par la société ENGELHARD sous le nom Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona), par la société Eckart sous la dénomination Prestige Bronze et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) et par la société Eckart sous la dénomination Prestige Copper ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), Dark Blue (117324) (Colorona), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges. En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.
On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.
La variété des pigments qui peuvent être utilisés dans la présente invention permet d'obtenir une riche palette de

couleurs, ainsi que des effets optiques particuliers tels que des effets métalliques, interférentiels.
La taille du pigment utilisé dans la composition cosmétique selon la présente invention est de préférence comprise entre 10 nm et 200 μm, de préférence entre 100 nm et 80 μm, préférentiellement entre 300 nm et 50 μm, et encore mieux entre 500 nm et 20 μm.

Les pigments peuvent être dispersés dans le produit grâce à un agent dispersant. L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750 g/mol tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.
Comme autre dispersant utilisable dans les compositions de l'invention, on peut citer les dérivés ammonium quaternaire d'acides gras polycondensés comme le Solsperse 17 000 vendu par la société Avecia et les mélanges de polydiméthylsiloxane/oxypropylène tels que ceux vendus par la société Dow Corning sous les références DC2-5185, DC2-5225 C, ou par la société Shin-Etsu sous la référence KF6017.

Les pigments susceptibles d'être utilisés dans la composition cosmétique selon l'invention peuvent être traités en surface par un agent organique. Ces pigments sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition selon l'invention. Ces agents organiques peuvent être par exemple choisis parmi les acides aminés; les cires, par exemple la cire de carnauba et la cire d'abeille; les acides gras, les alcools gras et leurs dérivés, tels que l'acide stéarique, l'acide hydroxystéarique, l'alcool stéarylique, l'alcool hydroxystéarylique, l'acide laurique et leurs dérivés; les tensio-actifs anioniques; les lécithines; les sels de sodium, potassium, magnésium, fer, titane, zinc ou aluminium d'acides gras, par exemple le stéarate ou laurate d'aluminium; les alcoxydes métalliques; les polysaccharides, par exemple le chitosane, la cellulose et ses dérivés; le polyéthylène; les polymères (méth)acryliques, par exemple les polyméthyl(méth)acrylates; les polymères et copolymères contenant des motifs acrylates; les protéines; les alcanolamines; les composés siliconés, par exemple les silicones, les polydiméthylsiloxanes, les alcoxysilanes, les alkylsilanes, les siloxysilicates; les composés organiques fluorés, par exemple les perfluoroalkyle éthers; les composés fluoro-siliconés. Les pigments traités en surface peuvent aussi avoir été traités par un mélange de ces composés et/ou avoir subi plusieurs traitements de surface. Les pigments traités en surface peuvent être préparés selon des techniques de traitement de surface bien connues de l'homme de l'art ou trouvés tels quels dans le commerce. De préférence, les pigments traités en surface sont recouverts par une couche organique. L'agent organique avec lequel sont traités les pigments peut être déposé sur les pigments par évaporation de solvant, réaction chimique entre les molécules de l'agent de surface ou création d'une liaison covalente entre l'agent de surface et les pigments. Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4578266. De préférence, on utilisera un agent organique lié aux pigments de manière covalente. L'agent pour le traitement de surface peut représenter de 0,1 à 50% en poids du poids total des pigments traités en surface, de préférence de 0,5 à 30% en poids, et encore plus préférentiellement de 1 à 10% en poids. De préférence, les traitements en surface des pigments sont choisis parmi les traitements suivants :

- un traitement PEG-silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone/Triméthylsiloxysilicate comme le traitement de surface Covasil 4.05 commercialisé par LCW ;
- un traitement LauroylLysine comme le traitement de surface LL commercialisé par LCW ;
- un traitement Perfluoropolyméthylisopropyl éther comme le traitement de surface FHC commercialisé par LCW ;
- un traitement Disodium Stéaroyl Glutamate comme le traitement de surface NAI commercialisé par Miyoshi ;
- un traitement Phosphate de Perfluoroalkyle comme le traitement de surface PF commercialisé par Daito ;
- un traitement Copolymère acrylate/Diméthicone et Phosphate de Perfluoalkyle comme le traitement de surface FSA commercialisé par Daito ;
- un traitement Octyltriéthylsilane comme le traitement de surface OTS commercialisé par Daito ;

- un traitement Copolymère Acrylate/Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement copolymère Acrylate comme le traitement de surface APD commercialisé par Daito.

Parmi les colorants susceptibles d'être utilisés, on peut citer les colorants synthétiques connus sous les dénominations D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), FD&C Yellow 5 (CI 19 140), FD&C Yellow 6 (CI 15 985), D&C Green 5 (CI 61 570), D&C Yellow 10 (CI 77 002), FD&C Green 3 (CI 42 053), FD&C Blue 1 (CI 42 090). On peut aussi citer les colorants naturels comme le caramel, le jus de betterave, la chlorophylline cuivrée, les anthocyanes, le lycopène et le béta-carotène.

De préférence, la quantité de matières colorantes dans la composition de maquillage est comprise entre 0,001 et 50% en poids, notamment 0,01 et 30% en poids, voire 0,025 et 20% en poids, du poids total de la composition.

La composition de maquillage selon l'invention peut en outre comprendre une ou plusieurs charge(s), qui peuvent être présentes à raison de 0,01 à 35% en poids, de préférence 0,1 à 20% en poids, du poids total de la composition. On peut citer le talc, le mica, la silice, le kaolin, le carbonate de calcium, le sulfate de baryum, les poudres de Nylon (Orgasol notamment) et de polyéthylène, le Téflon, l'amidon, le nitrure de bore, des microsphères de copolymères telles que l'Expancel (Nobel Industrie), et les microbilles de résine de silicone (Tospearl de Toshiba, par exemple) ; ainsi que leurs mélanges.

La composition selon l'invention peut comprendre une phase grasse, qui peut représenter 1 à 98% en poids, notamment 5 à 95% en poids, voire 10 à 90% en poids, du poids total de la composition. Cette phase grasse peut comprendre des huiles, des cires et/ou des composés pâteux.

La composition selon l'invention peut donc comprendre une ou plusieurs huiles, qui peuvent être choisies parmi les huiles hydrocarbonées, les huiles siliconées, les huiles fluorées et leurs mélanges. Les huiles peuvent être d'origine animale, végétale, minérale ou synthétique. Les huiles peuvent être volatiles ou non volatiles. On entend par huile, tout corps gras sous forme liquide à température ambiante (20-25°C) et à pression atmosphérique.

Par "huile volatile", on entend au sens de l'invention toute huile susceptible de s'évaporer au contact des matières kératiniques en moins d'une heure, à température ambiante et pression atmosphérique. Les huiles volatiles ont de préférence une pression de vapeur non nulle, à température ambiante et pression atmosphérique, allant de 0,13 Pa à 40 000 Pa, en particulier de 1,3 Pa à 13 000 Pa, plus particulièrement de 1,3 Pa à 1300 Pa.

On entend par "huile siliconée", une huile comprenant au moins un atome de silicium, et notamment au moins un groupe Si-O.

On entend par "huile fluorée", une huile comprenant au moins un atome de fluor. On entend par "huile hydrocarbonée", une huile contenant principalement des atomes d'hydrogène et de carbone.

Les huiles peuvent éventuellement comprendre des atomes d'oxygène, d'azote, de soufre et/ou de phosphore, par exemple, sous la forme de radicaux hydroxyle ou acide.

Les huiles volatiles peuvent être choisies parmi les huiles hydrocarbonées ayant de 8 à 16 atomes de carbone, et notamment les alcanes ramifiés en $C_8$-$C_{16}$ (appelées aussi isoparaffines), comme l'isododécane, l'isodécane, l'isohexadécane.

L'huile hydrocarbonée volatile peut également être un alcane volatil linéaire comprenant 7 à 17 atomes de carbone, en particulier 9 à 15 atomes de carbone, et plus particulièrement 11 à 13 atomes de carbone. On peut notamment citer le n-nonadécane, le n-décane, le n-undécane, le n-dodécane, le n-tridécane, le n-tétradécane, le n-pentadécane, le n-hexadécane, et leurs mélanges.

Comme huiles volatiles, on peut aussi utiliser les silicones volatiles, et notamment les huiles de silicones linéaires ou cycliques volatiles, en particulier celles ayant une viscosité inférieure ou égale à 8 centistokes ($8.10^{-6}m^2/s$), et ayant notamment 2 à 10 atomes de silicium, en particulier 2 à 7 atomes de silicium, ces silicones comportant éventuellement des groupes alkyle ou alkoxy ayant 1 à 10 atomes de carbone. On peut citer les diméthicones de viscosité 5 et 6 cSt, l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, et leurs mélanges.

Comme huile non volatile, on peut notamment citer :

- les huiles hydrocarbonées d'origine animale,
- les huiles hydrocarbonées d'origine végétale, telles que les esters de phytostéaryle, tels que l'oléate de phytostéaryle, l'isostéarate de phytostéaryle et le glutamate de lauroyl/octyldodécyle/phytostéaryle; les triglycérides constitués d'esters d'acides gras et de glycérol, en particulier, dont les acides gras peuvent avoir des longueurs de chaînes variant de $C_4$ à $C_{36}$, et, notamment, de $C_{18}$ à $C_{36}$, ces huiles pouvant être linéaires ou ramifiées, saturées ou

insaturées; ces huiles peuvent, notamment, être des triglycérides heptanoïques ou octanoïques, l'huile de karité, de luzerne, de pavot, de potimarron, de millet, d'orge, de quinoa, de seigle, de bancoulier, de passiflore, le beurre de karité, l'huile d'aloès, l'huile d'amande douce, l'huile d'amande de pêche, l'huile d'arachide, l'huile d'argan, l'huile d'avocat, l'huile de baobab, l'huile de bourrache, l'huile de brocoli, l'huile de calendula, l'huile de caméline, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de lys, l'huile de macadamia, l'huile de maïs, l'huile de meadowfoam, l'huile de millepertuis, l'huile de monoï, l'huile de noisette, l'huile de noyaux d'abricot, l'huile de noix, l'huile d'olive, l'huile d'onagre, l'huile de palme, l'huile de pépins de cassis, l'huile de pépins de kiwi, l'huile de pépins de raisin, l'huile de pistache, l'huile de potimarron, l'huile de potiron, l'huile de quinoa, l'huile de rosier muscat, l'huile de sésame, l'huile de soja, l'huile de tournesol, l'huile de ricin, et l'huile de watermelon, et leurs mélanges, ou encore des triglycérides d'acides caprylique/caprique, comme ceux vendus par la société Stéarineries Dubois ou ceux vendus sous les dénominations Miglyol 810®, 812® et 818® par la société Dynamit Nobel,

- les éthers de synthèse ayant de 10 à 40 atomes de carbone ;
- les esters de synthèse, comme les huiles de formule $R_1COOR_2$, dans laquelle $R_1$ représente un reste d'un acide gras linéaire ou ramifié comportant de 1 à 40 atomes de carbone et $R_2$ représente une chaîne hydrocarbonée, notamment, ramifiée contenant de 1 à 40 atomes de carbone à condition que $R_1$ + $R_2$ soit supérieur ou égal à 10. Les esters peuvent être, notamment, choisis parmi les esters d'alcool et d'acide gras, comme par exemple l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés, comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools, comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en $C_{12}$-$C_{15}$, le laurate d'hexyle, les esters de l'acide néopentanoïque, comme le néopentanoate d'isodécyle, le néopentanoate d'isotridécyle, le néopentanoate d'isostéaryle, le néopentanoate d'octyldodécyle, les esters de l'acide isononanoïque, comme l'isononanoate d'isononyle, l'isononanoate d'isotridécyle, l'isononanoate d'octyle, les esters hydroxylés comme le lactate d'isostéaryle, le malate de di-isostéaryle ;
- les esters de polyols et les esters du pentaérythritol, comme le tétrahydroxystéarate/tétraisostéarate de dipentaérythritol,
- les esters de dimères diols et de dimères diacides,
- les copolymères de dimère diol et de dimère diacide et leurs esters, tels que les copolymères dimères dilinoleyl diol/dimères dilinoléiques et leurs esters,
- les copolymères de polyols et de dimères diacides, et leurs esters,
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique, le 2-hexyldécanol, le 2-butyloctanol, et le 2-undécylpentadécanol,
- les acides gras supérieurs en $C_{12}$-$C_{22}$, tels que l'acide oléique, l'acide linoléique, l'acide linolénique et leurs mélanges;
- les carbonates de di-alkyle, les 2 chaînes alkyles pouvant être identiques ou différentes, tels que le dicaprylyl carbonate ;
- les huiles de masse molaire comprise entre environ 400 à environ 10 000 g/mol, en particulier environ 650 à environ 10 000 g/mol, en particulier, d'environ 750 à environ 7500 g/mol, et plus particulièrement variant d'environ 1000 à environ 5000 g/mol; on peut notamment citer, seuls ou en mélange, (i) les polymères lipophiles tels que les polybutylènes, les polyisobutylènes, par exemple, hydrogénés, les polydécènes et les polydécènes hydrogénés, les copolymères de la vinylpyrrolidone, tels que le copolymère vinylpyrrolidone/1-hexadécène, et les copolymères de polyvinylpyrrolidone (PVP), tels que les copolymères d'un alcène en $C_2$-$C_{30}$, tel qu'en $C_3$-$C_{22}$, et des associations de ceux-ci; (ii) les esters d'acides gras linéaires ayant un nombre total de carbone allant de 35 à 70, comme le tétrapélargonate de pentaérythrityle; (iii) les esters hydroxylés tels que le triisostéarate de polyglycérol-2; (iv) les esters aromatiques tels que le tridécyl trimellitate; (v) les esters d'alcools gras ou d'acides gras ramifiés en $C_{24}$-$C_{28}$, tels que ceux décrits dans le brevet US 6,491,927 et les esters du pentaérythritol, et, notamment, le citrate de triisoarachidyle, le tétraisononanoate de pentaérythrityle, le triisostéarate de glycéryle, le tri décyl-2 tétradécanoate de glycéryle, le tétraisostéarate de pentaérythrityle, le tétraisostéarate de polyglycéryle-2 ou encore le tétra décyl-2 tétradécanoate de pentaérythrityle; (vi) les esters et polyesters de dimère diol, tels que les esters de dimère diol et d'acide gras, et les esters de dimère diols et de diacide; (vii) les huiles siliconées, telles que les silicones phénylées; les polydiméthylsiloxanes (PDMS) non volatiles, les PDMS comportant des groupements alkyle ou alcoxy pendants et/ou en bouts de chaîne siliconée, groupements ayant chacun de 2 à 24 atomes de carbone, les silicones phénylées, comme les phényl triméthicones, les phényl diméthicones, les phényl triméthylsiloxy diphénylsiloxanes, les diphényl diméthicones, les diphényl méthyldiphényl trisiloxanes, et les 2-phényléthyltriméthylsiloxysilicates, les diméthicones

ou phényltriméthicone de viscosité inférieure ou égale à 100 cSt.

De préférence, la quantité d'huile(s) dans la composition de maquillage selon l'invention est comprise entre 1 et 90% en poids, notamment 2 à 75% en poids, voire 3 à 60% en poids, du poids total de la composition.

La composition selon l'invention peut également comprendre au moins une cire et/ou un pâteux. De préférence, la quantité de cires dans la composition de maquillage selon l'invention est comprise entre 0,5 et 30% en poids, notamment 1 à 20% en poids, voire 2 à 15% en poids, du poids total de la composition. De préférence, la quantité de pâteux dans la composition de maquillage selon l'invention est comprise entre 0,5 et 50% en poids, notamment 1 à 40% en poids, voire 2 à 30% en poids, du poids total de la composition.

Par "cire", on entend un composé lipophile, solide à température ambiante (25°C), à changement d'état solide/liquide réversible, ayant un point de fusion supérieur ou égal à 30°C pouvant aller jusqu'à 200°C. Les cires peuvent être choisies parmi les cires d'origine animale, végétale, minérale ou de synthèse et leurs mélanges. On peut notamment citer les cires hydrocarbonées comme la cire d'abeilles, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters. On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de tournesol hydrogénée, l'huile de ricin hydrogénée, l'huile de coprah hydrogénée, l'huile de lanoline hydrogénée, et le tétrastéarate de di-(triméthylol-1,1,1 propane). On peut encore citer les cires de silicone, les cires fluorées. On peut également utiliser les cires obtenues par hydrogénation d'huile de ricin estérifiée avec l'alcool cétylique.

Par "pâteux", on entend un composé gras lipophile à changement d'état solide/liquide réversible et comportant à la température de 23°C une fraction liquide et une fraction solide. Le composé pâteux a de préférence une dureté à 20°C allant de 0,001 à 0,5 MPa, de préférence de 0,002 à 0,4 MPa. Le composé pâteux est de préférence choisi parmi les composés synthétiques et les composés d'origine végétale. Un composé pâteux peut être obtenu par synthèse à partir de produits de départ d'origine végétale. On peut notamment citer, seul ou en mélange, :

- la lanoline et ses dérivés, tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non, comme les polydiméthylsiloxanes de masses moléculaires élevées, les polydiméthylsiloxanes à chaînes latérales du type alkyle ou alcoxy ayant de 8 à 24 atomes de carbone, notamment les stéaryl diméthicones,
- les composés fluorés polymères ou non,
- les polymères vinyliques, notamment les homopolymères d'oléfines; les copolymères d'oléfines; les homopolymères et copolymères de diènes hydrogénés; les oligomères linéaires ou ramifiés, homo ou copolymères de (méth)acrylates d'alkyles ayant de préférence un groupement alkyle en $C_8$-$C_{30}$ ; les oligomères homo et copolymères d'esters vinyliques ayant des groupements alkyles en C8-C30 ; les oligomères homo et copolymères de vinyléthers ayant des groupements alkyles en C8-C30;
- les polyéthers liposolubles résultant de la polyéthérification entre un ou plusieurs diols en $C_2$-$C_{100}$, de préférence en $C_2$-$C_{50}$ ; et notamment les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30;
- les éthers de polyol choisis parmi les éthers de pentaérythritol et de polyalkylène glycol, les éthers d'alcool gras et de sucre, et leurs mélanges, l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges;
- les esters et les polyesters; et notamment (i) les esters d'un glycérol oligomère, notamment les esters de diglycérol, en particulier les condensats d'acide adipique et de glycérol, pour lesquels une partie des groupes hydroxyles des glycérols a réagi avec un mélange d'acides gras tels que l'acide stéarique, l'acide caprique, l'acide stéarique et l'acide isostéarique et l'acide 12-hydroxystéarique; (ii) les esters de phytostérol, (iii) les esters de pentaérythritol; (iv) les esters formés à partir d'au moins un alcool, l'un au moins des alcools étant un alcool de Guerbet et d'un dimère diacide formé à partir d'au moins un acide gras insaturé; (v) les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$, (vi) les polyesters qui résultent de l'estérification, par un acide polycarboxylique d'un ester d'acide hydroxycarboxylique aliphatique; (vii) les esters aliphatiques d'ester résultant de l'estérification d'un ester d'acide hydroxycarboxylique aliphatique par un acide carboxylique aliphatique comprenant notamment 4 à 30 atomes de carbone. L'ester d'acide hydroxycarboxylique aliphatique est avantageusement issu d'un acide carboxylique aliphatique hydroxylé comportant 2 à 40 atomes de carbone et 1 à 20 groupes hydroxyle; (viii) les esters

aliphatiques d'ester choisis parmi l'ester résultant de la réaction d'estérification de l'huile de ricin hydrogénée avec l'acide isostéarique (mono-, di- ou tri-isostéarate d'huile de ricin hydrogénée).

Le composé pâteux peut également être d'origine végétale. On peut notamment citer l'huile de jojoba isomérisée telle que l'huile de jojoba partiellement hydrogénée isomérisée trans; la cire d'orange, le beurre de karité, l'huile d'olive partiellement hydrogénée, le beurre de cacao, l'huile de mangue.

La composition selon l'invention peut également comprendre une phase aqueuse, qui peut représenter 1 à 80% en poids, notamment 2 à 70% en poids, voire 3 à 60% en poids, du poids total de la composition. Cette phase aqueuse peut être constituée essentiellement d'eau, ou peut comprendre un mélange d'eau et de solvant miscible à l'eau (miscibilité dans l'eau supérieure à 50% en poids à 25°C) notamment choisi parmi les monoalcools ayant 1 à 5 atomes de carbone tels que l'éthanol, l'isopropanol, les glycols ayant 2 à 8 atomes de carbone tels que le propylène glycol, l'éthylène glycol, le 1,3-butylène glycol, le dipropylène glycol, les cétones en C3-C4, les aldéhydes en C2-C4 et leur mélanges.

La composition selon l'invention peut également comprendre au moins un tensioactif, qui peut être présent à raison de 0,1 à 10% en poids, notamment 0,5 à 8% en poids, voire 1 à 6% en poids, du poids total de la composition. Le tensioactif peut être choisi parmi les agents tensioactifs amphotères, anioniques, cationiques ou non ioniques, de préférence non ioniques. On peut notamment citer, seuls ou en mélange :

a) les agents tensioactif non ioniques de HLB inférieur à 8 à 25°C, éventuellement associés à un ou plusieurs agents tensioactif non ioniques de HLB supérieur à 8 à 25°C tels que cités plus bas, comme :

- les esters et éthers d'oses tels que les stéarates de saccharose, cocoate de saccharose, stéarate de sorbitan et leurs mélanges;
- les esters d'acides gras, notamment en $C_8$-$C_{24}$, et de préférence en $C_{16}$-$C_{22}$, et de polyol, notamment de glycérol ou de sorbitol, tels que le stéarate de glycéryle, le laurate de glycéryle, le stéarate de polyglycéryle-2, le tristéarate de sorbitan, et le ricinoléate de glycéryle ;
- les lécithines, telles que les lécithines de soja;
- les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 2 motifs oxyéthylène (nom CTFA : Steareth-2) ;
- les tensioactifs siliconés comme les diméthicones copolyols et les alkyldiméthicones copolyols, et par exemple le mélange de cyclométhicone/diméthicone copolyol vendu sous la dénomination Q2-3225C® par la société DOW CORNING ;

b) les agents tensioactifs non ioniques de HLB supérieur ou égal à 8 à 25°C, comme :

- les esters et éthers d'oses tels que le mélange de cétylstéaryl glucoside et d'alcools cétylique et stéarylique comme le Montanov 68 de Seppic;
- les éthers de glycérol, oxyéthylénés et/ou oxypropylénés, pouvant comporter de 1 à 150 motifs oxyéthylène et/ou oxypropylène ;
- les éthers oxyéthylénés et/ou oxypropylénés (pouvant comporter de 1 à 150 motifs oxyéthylène et/ou oxypropylène) d'alcools gras, notamment en $C_8$-$C_{24}$, et de préférence en $C_{12}$-$C_{18}$, tels que l'éther oxyéthyléné de l'alcool stéarylique à 20 motifs oxyéthylène (nom CTFA : Steareth-20), l'éther oxyéthyléné de l'alcool cétéarylique à 30 motifs oxyéthylène (Ceteareth-30) et l'éther oxyéthyléné du mélange d'alcools gras en $C_{12}$-$C_{15}$ comportant 7 motifs oxyéthylène ($C_{12-15}$ Pareth-7);
- les esters d'acide gras, notamment en $C_8$-$C_{24}$, et de préférence en $C_{16}$-$C_{22}$, et de polyéthylène glycol (ou PEG) (pouvant comprendre de 1 à 150 motifs oxyéthylène), tels que le stéarate de PEG-50 et le monostéarate de PEG-40;
- les esters d'acide gras, notamment en $C_8$-$C_{24}$, et de préférence en $C_{16}$-$C_{22}$, et d'éthers de glycérol oxyéthylénés et/ou oxypropylénés (pouvant comporter de 1 à 150 motifs oxyéthylène et/ou oxypropylène), comme le monostéarate de glycéryle polyoxyéthyléné à 200 motifs oxyéthylène, le stéarate de glycéryle polyoxyéthyléné à 30 motifs oxyéthylène, l'oléate de glycéryle polyoxyéthyléné à 30 motifs oxyéthylène, le cocoate de glycéryle polyoxyéthyléné à 30 motifs oxyéthylène, l'isostéarate de glycéryle polyoxyéthyléné à 30 motifs oxyéthylène et le laurate de glycéryle polyoxyéthyléné à 30 motifs oxyéthylène,
- les esters d'acide gras, notamment en $C_8$-$C_{24}$, et de préférence en $C_{16}$-$C_{22}$, et d'éthers de sorbitol oxyéthylénés et/ou oxypropylénés (pouvant comporter de 1 à 150 motifs oxyéthylène et/ou oxypropylène), comme le polysorbate 20 et le polysorbate 60;
- le diméthicone copolyol, tel que le Q2-5220® de DOW CORNING ;

- le diméthicone copolyol benzoate tel que les FINSOLV SLB 101® et 201® de FINETEX ;
- les copolymères d'oxyde de propylène et d'oxyde d'éthylène, également appelés polycondensats OE/OP, qui sont des copolymères consistant en des blocs polyéthylène glycol et polypropylène glycol, comme par exemple les polycondensats tribloc polyéthylène glycol/polypropylène glycol/polyéthylène glycol.

c) les tensioactifs anioniques tels que :

- les sels d'acides gras en $C_{16}$-$C_{30}$, notamment les sels aminés comme le stéarate de triéthanolamine ou le stéarate de 2-amino-2-méthylpropane-1,3-diol ;
- les sels d'acides gras polyoxyéthylénés, notamment les sels aminés ou les sels de métaux alcalins, et leurs mélanges ;
- les esters phosphoriques et leurs sels tels que le «DEA oleth-10 phosphate» (Crodafos N 10N de la société CRODA) ou le phosphate de monocétyle monopotassique;
- les sulfosuccinates tels que le « Disodium PEG-5 citrate lauryl sulfosuccinate » et le « Disodium ricinoleamido MEA sulfosuccinate » ;
- les alkyléthersulfates tels que le lauryl éther sulfate de sodium ;
- les iséthionates ;
- les acylglutamates tels que le «Disodium hydrogenated tallow glutamate» (AMISOFT HS-21 R® d'AJINOMOTO) et le sodium stearoyl glutamate (AMISOFT HS-11 PF® d'AJINOMOTO);
- les dérivés de soja comme le potassium soyate ;
- les citrates, comme le glycéryl stéarate citrate;
- les dérivés de proline, comme le sodium palmitoyl proline ou le mélange de sodium palmitoyl sarcosinate, magnésium palmitoyl glutamate, acide palmitique et palmitoyl proline (Sepifeel One de Seppic) ;
- les lactylates, comme le sodium stéaroyl lactylate ;
- les sarcosinates, comme le sodium palmitoyl sarcosinate ou le mélange de stéaroyl sarcosine et myristoyl sarcosine 75/25;
- les sulfonates, comme le sodium $C_{14-17}$ alkyl-sec-sulfonate;
- les glycinates, comme le sodium cocoyl glycinate.

d) les tensioactifs cationiques, tels que :

- les alkylimidazolidiniums tels que l'étho-sulfate d'isostéaryl-éthylimidonium,
- les sels d'ammonium tels que les halogénures d'(alkyl en $C_{12-30}$)-tri(alkyl en $C_{1-4}$) ammonium comme le chlorure de N,N,N-triméthyl-1-docosanaminium (ou chlorure de Behentrimonium).

e) les tensioactifs amphotères comme les N-acyl-aminoacides tels que les N-alkyl-aminoacétates et le cocoamphodiacétate disodique, et les oxydes d'amines tels que l'oxyde de stéaramine.

La composition de maquillage selon l'invention peut également comprendre au moins un agent utilisé habituellement en cosmétique choisi, par exemple, parmi des agents réducteurs, des adoucissants, des agents anti-mousse, des agents hydratants, des filtres UV, des céramides; des actifs cosmétiques; des peptisants, des parfums, des protéines, des vitamines, des propulseurs, des polymères filmogènes ou non, hydrophiles ou lipophiles; des gélifiants lipophiles ou hydrophiles. Les additifs ci-dessus sont en général présents en une quantité de 0,01 à 10% en poids, pour chacun d'eux, par rapport au poids total de la composition. Bien entendu, l'homme de l'art veillera à choisir les constituants de la composition de manière telle que les propriétés avantageuses attachées à l'invention ne soient pas, ou substantiellement pas, altérées.

La composition de maquillage selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte.
L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Dans un mode de réalisation particulier de l'invention, la composition peut se présenter sous forme d'une émulsion, notamment une émulsion huile-dans-eau ou eau-dans-huile ou multiple, de préférence eau-dans huile, comprenant de préférence (% en poids) :

- de 1 à 80% de phase aqueuse, pouvant comprendre éventuellement en plus de l'eau, des additifs comme des

polyols, des sels, des actifs ou encore des polymères hydrophiles,

- de 0,1 à 10% de tensioactif(s), de préférence non ionique,
- de 1 à 90% de phase grasse, pouvant comprendre éventuellement en plus des huiles, des cires, des pâteux, des actifs, ou des parfums,
- de 5 à 20% de matière(s) colorante(s),
- de 0,1 à 20% de charge(s) et
- de 0,1 à 10% de polymère(s) selon l'invention.

Une telle composition sous forme d'émulsion trouve une application tout particulière en tant que fond de teint.

Dans un autre mode de réalisation particulier de l'invention, la composition peut être anhydre et notamment se présenter sous forme d'un stick ou bâton, comprenant de préférence (% en poids) :

- de 1 à 20% de matière(s) colorante(s),
- de 1 à 50% de cire(s),
- de 1 à 30% de pâteux,
- de 1 à 60% d'huile(s),
- de 0,1 à 10% de charge(s),
- de 0,1 à 70% de polymère(s) selon l'invention.

Une telle composition sous forme de stick ou bâton trouve une application tout particulière en tant que rouge à lèvres.

La composition selon l'invention se présente donc sous forme de composition de maquillage de la peau et/ou des lèvres, notamment de la peau du visage ou du corps; elle peut être un produit pour le teint tel qu'un fond de teint, un fard à joues ou à paupières; un produit pour les lèvres tel qu'un rouge à lèvres ou un soin des lèvres; un produit anticernes; un blush, un eye-liner; un crayon à lèvres ou à yeux; un produit de maquillage du corps; un gloss (brillant à lèvres).

L'invention est illustrée plus en détail dans les exemples qui suivent.

**Méthode de mesure de l'indice de transfert**

A/

L'indice de transfert en présence de sébum du dépôt obtenu avec une composition est déterminé selon le protocole de mesure décrit ci-après.

On prépare un support (carré de 40 mm x 40 mm) constitué d'une couche de mousse de néoprène adhésif sur une de ses faces (vendue sous la dénomination RE40X70 RE70X40 212B de la société JOINT TECHNIQUE LYONNAIS Ind.). Sur la face non adhésive du support, on fixe une couronne adhésive ayant un diamètre interne de 24 mm et dont l'épaisseur est d'environ 250 μm. On applique à l'intérieur de la couronne la composition que l'on arase avec une lame de verre pour obtenir un dépôt de la composition d'environ 250 μm d'épaisseur, puis on retire la couronne et on laisse sécher pendant 20 heures à l'étuve à 37°C.

Le support est ensuite collé par sa face adhésive sur un embout d'un diamètre de 27 mm fixé sur une presse (STATIF MANUEL SV-1 de la société IMADA Co LTD) équipée d'un dynanomètre (DPS-5R de la société IMADA Co LTD).

Sur un papier couché qualité photo (référence EPSON S041061 de 102g/$m^2$), on dessine une bande de 4 cm de largeur et 21 cm de longueur; dans cette bande, on dessine 5 cases ayant chacune une longueur de 4,2 cm selon l'axe longitudinal de la bande. Le papier est placé sur le socle de la presse.

On dépose au centre de la première case une goutte de 10 μl de sébum artificiel ayant la composition suivante :

- trioléine 29 %
- acide oléïque 28,5 %
- oléate d'oléyle 18,5%
- squalène 14%
- cholestérol 7 %
- palmitate de cholestérol 3 %

Puis on presse le support (comportant l'échantillon de composition) sur la première case de la bande de papier à une force d'environ 4 kg exercée pendant 5 secondes. On déplace de manière rectiligne et régulière le papier sur toute la longueur de la bande de telle sorte que le support soit en contact avec toute la longueur de la bande. La vitesse de déplacement de la bande est de l'ordre de 10 cm/s.

On observe visuellement la traînée de produit déposée sur la bande de papier. On attribue une note allant

respectivement de 0,5 à 5 par incrément de 0,5 en fonction du nombre de cases, de la première à la cinquième, traversées en tout ou partie par l'éventuelle traînée de produit.

La note 5 est attribuée dans le cas où la trainée de produit va jusqu'au bout de la cinquième case, voire au delà. La note 5 est attribuée lorsqu'à l'observation, après avoir fait le déplacement relatif entre le papier et le support, il ne subsiste sur le support sensiblement aucun produit (moins de 10%). Dans ce dernier cas, le transfert peut être qualifié de total. La note 5 est également attribuée lorsque la traînée de produit s'étend au delà de la cinquième case, indépendamment de la quantité de produit subsistant sur le support.

La note 0 est attribuée dans le cas où aucun produit présent sur le support n'est transféré sur la bande de papier. Aucune trace visible ne peut être observée sur la feuille. Le transfert peut être qualifié de nul.

Par convention, le trait de séparation entre la case n et la case n+1 fait partie de la case n.

Le tableau ci dessous illustre la façon dont sont attribuées les autres notes en fonction de l'endroit sur les cases 1 à 5 où s'arrête la traînée de produit. Pour ces notes, il reste une quantité plus ou moins grande de produit sur le support. Le transfert est partiel.

| | Note | |
|---|---|---|
| N° de la case où s'arrête la trainée de produit | Au delà de la moitié de la case | Au plus à la moitié de la case |
| 5 | 4,5 | 4 |
| 4 | 3,5 | 3 |
| 3 | 2,5 | 2 |
| 2 | 1,5 | 1 |
| 1 | 0,5 | |

B/

L'indice de transfert en présence de sueur du dépôt obtenu avec une composition est déterminé selon le même protocole de mesure, en remplaçant la goutte de 10 µl de sébum artificiel, par une goutte de 10 µl de sueur artificielle ayant la composition suivante :

- acide lactique 0,1142 %
- Ammoniaque 0,1764%
- Glycine 0,00758 %
- Serine 0,0214%
- Urée 0,051 %
- Chlorure de sodium 0,07949%
- Eau pure qsp 100 %

**Exemple 1 : synthèse du polymère 1 selon l'invention**

On sèche 100 g de polymère 1,2-polybutadiène hydrogéné dihydroxylé (GI3000 de la société Nisso; Mn=3100)

à 80°C, sous pression réduite, pendant une nuit. Ce polymère est mis en solution dans 400 ml de toluène anhydre. On ajoute 25 μl de catalyseur (dilaurate de dibutylétain) et on chauffe à 80°C, sous agitation, jusqu'à obtention d'une solution homogène. On ajoute 15 g de molécule fonctionnalisée isocyanate de structure suivante :

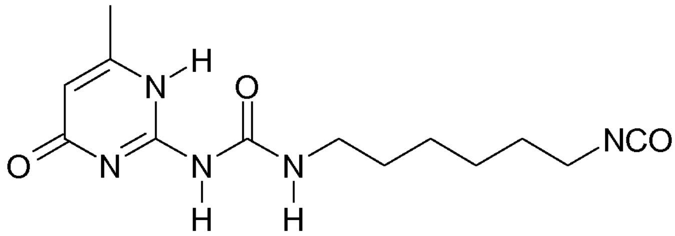

en solution dans 300 ml de toluène anhydre, sous atmosphère contrôlée à 40°C. Le mélange réactionnel est chauffé à 100°C et agité à cette température pendant 4 heures. Le suivi de la réaction est fait par spectroscopie infrarouge, avec le suivi de la disparition totale du pic caractéristique des isocyanates à 2260 $cm^{-1}$. A la fin de la réaction, on ajoute 100 ml d'éthanol pour éliminer toute trace d'isocyanate résiduel, puis on filtre le mélange après avoir rajouté de l'isododécane pour rendre la solution moins visqueuse. La solution de polymère est alors directement strippée à l'isododécane.

On obtient une solution du polymère final dans l'isododécane, à 21% d'extrait sec; le polymère est caractérisé par GPC (Mn = 6400 et indice de polydispersité = 1,85) et RMN 1H (spectre conforme à ce qui est attendu).

**Exemple 2 : synthèse du polymère 2 selon l'invention**

On chauffe 106,1 g de polymère 1,2-polybutadiène hydrogéné dihydroxylé (GI2000 de Nisso, Mn=2100), en présence de 22 mg de catalyseur (dilaurate de dibutylétain) à 80°C, sous pression réduite, pendant 2 heures. La température du mélange est descendue à 20°C, sous argon, suivi de l'addition de 10 ml d'isododécane et de 19,3 g d'isophorone diisocyanate (IPDI). Le mélange est agité pendant 16 heures à 20°C, sous atmosphère contrôlée, puis est chauffé à 120°C, suivi de l'addition de 25 ml de propylène carbonate. On ajoute 12 g de 6-méthyl isocytosine, résultant en une suspension homogène blanche. Cette suspension est chauffée à 140°C et est agitée à cette température pendant 6 heures. La réaction est suivie par spectroscopie infrarouge, jusqu'à la disparition totale du pic caractéristique des isocyanates (2250 $cm^{-1}$). Le mélange est ensuite redescendu à 30°C, et on y ajoute 400 ml d'heptane, 200 ml de THF et 50 ml d'éthanol, avant filtration sur célite. Le mélange est alors strippé à l'isododécane.

On obtient au final une solution du polymère dans l'isododécane, à 20% d'extrait sec; le polymère est caractérisé par GPC (Mn = 7000 et indice de polydispersité = 2,05).

**Exemple 3 : synthèse du polymère 3 selon l'invention**

On chauffe 99 g de polymère 1,2-polybutadiène hydrogéné dihydroxylé (GI3000 de Nisso, Mn=3100), en présence de 22 mg de catalyseur (dilaurate de dibutylétain) à 80°C, sous pression réduite, pendant 2 heures. La température du mélange est descendue à 20°C, sous argon, suivi de l'addition de 30 ml d'isododécane et de 11 g d'isophorone diisocyanate (IPDI). Le mélange est agité pendant 16 heures à 20°C, sous atmosphère contrôlée, puis est chauffé à 120°C, suivi de l'addition de 25 ml de propylène carbonate. On ajoute 8,1 g de 6-méthyl isocytosine, résultant en une suspension homogène blanche. Cette suspension est chauffée à 140°C et est agitée à cette température pendant 6 heures. La réaction est suivie par spectroscopie infrarouge, jusqu'à la disparition totale du pic caractéristique des isocyanates (2250 $cm^{-1}$). Le mélange est ensuite redescendu à 30°C, et on y ajoute 1 litre d'heptane, avant filtration sur célite. Le mélange est alors strippé à l'isododécane.

On obtient au final une solution du polymère dans l'isododécane, à 20% d'extrait sec; le polymère est caractérisé par GPC (Mn = 4200 et indice de polydispersité = 2,34).

**Exemple 4 : synthèse du Polymère 4 selon l'invention**

On chauffe 89 g de polymère 1,2-polybutadiène hydrogéné dihydroxylé (GI3000 de Nisso, Mn=3100), en présence de 22 mg de catalyseur (dilaurate de dibutylétain) à 80°C, sous pression réduite, pendant 2 heures. La température du mélange est descendue à 20°C, sous argon, suivi de l'addition de 60 ml d'isododécane et de 11,6 g de 4,4'-dicyclohexylméthane diisocyanate. Le mélange est agité pendant 16 heures à 20°C, sous atmosphère contrôlée, puis est chauffé à 120°C, suivi de l'addition de 40 ml de propylène carbonate. On ajoute 6,64 g de 6-méthyl isocytosine, résultant en une suspension homogène blanche. Cette suspension est chauffée à 140°C et est agitée à cette température pendant 8 heures. La réaction est suivie par spectroscopie infrarouge, jusqu'à la disparition totale du pic caractéristique

des isocyanates (2250 $cm^{-1}$). Le mélange est ensuite redescendu à 30°C, et on y ajoute 250 ml d'isododécane et 500 ml d'heptane, avant filtration sur célite.

Le mélange est alors strippé à l'isododécane.

On obtient au final une solution du polymère dans l'isododécane, à 22% d'extrait sec; le polymère est caractérisé par GPC (Mn = 10700 et indice de polydispersité = 2,26).

**Exemple 5 : synthèse du polymère 5 selon l'invention**

On chauffe 143,1 g de polymère 1,2-polybutadiène hydrogéné dihydroxylé (GI2000 de Nisso, Mn=2100), en présence de 33 mg de catalyseur (dilaurate de dibutylétain) à 80°C, sous pression réduite, pendant 2 heures. La température du mélange est descendue à 20°C, sous argon, suivi de l'addition de 85 ml d'isododécane et de 30,8 g de 4,4'-dicyclohexylméthane diisocyanate. Le mélange est agité pendant 16 heures à 20°C, sous atmosphère contrôlée, puis est chauffé à 120°C, suivi de l'addition de 70 ml de propylène carbonate. On ajoute 22,6 g de 6-méthyl isocytosine, résultant en une suspension homogène blanche. Cette suspension est chauffée à 140°C et est agitée à cette température pendant 8 heures. La réaction est suivie par spectroscopie infrarouge, jusqu'à la disparition totale du pic caractéristique des isocyanates (2250 $cm^{-1}$). Le mélange est ensuite redescendu à 20°C, et on y ajoute 700 ml d'isododécane et 500 ml d'heptane, avant filtration sur célite. Le mélange est alors strippé à l'isododécane.

On obtient au final une solution du polymère dans l'isododécane, à 20% d'extrait sec; le polymère est caractérisé par GPC (Mn = 8400 et indice de polydispersité = 2,00).

**Exemple 6 : Fond de teint selon l'invention**

On prépare un fond de teint liquide comprenant (% en poids):

Phase A1:

- Cetyl PEG/PPG-14/14 dimethicone (ABIL EM 97 de Goldschmidt) 1,8%
- Isostéaryl diglycéryle succinate 0,6%
- Isododécane 15,5%
- Cyclopentasiloxane 5,0%
- Polymère de l'exemple 1 (à 21 % dans isododécane) 10% (soit 2,1 % MS)

Phase A2:

- Cyclopentasiloxane 5,0%
- Oxydes de fer enrobés de stéaroyl glutamate d'aluminium 3,2%
- Dioxyde de titane enrobé de stéaroyl glutamate d'aluminium 6,8%

Phase A3:

- Gomme de silicone phénylée + cyclopentasiloxane (MIRASIL C-DPDM de Blue Star) 3,0%

Phase A4:

- Poudre de Nylon 12 8,0%

Phase B:

- conservateur q.s.
- Sulfate de magnésium 0,7%
- Eau qsp 100%

On pèse les constituants de la phase A1 dans un bécher, sous agitation au Moritz à 1500 tours/min en maintenant à température ambiante (25°C). La phase A2 est préparée séparément en broyant les pigments à la tricylindre puis elle est ajoutée sous agitation au mélange précédent. On ajoute ensuite les phases A3 et A4 en maintenant l'agitation. Pour préparer la phase B, on porte l'eau à l'ébullition puis on ajoute les autres constituants de la phase B. L'émulsion se fait à température ambiante. Les deux phases doivent avoir une température voisine de l'ambiante. On verse la phase

aqueuse B lentement dans la phase grasse en augmentant au fur et à mesure la vitesse d'agitation du Moritz jusqu'à 4000 tours/min. Après addition, on laisse agiter encore pendant 10 minutes à température ambiante.

On obtient un fond de teint liquide qui présente une bonne tenue.

**Exemple 7 : comparatif**

1/ On prépare un fond de teint liquide comparatif comprenant (% en poids) :

Phase A1:

- Cetyl PEG/PPG-14/14 dimethicone (ABIL EM 97 de Goldschmidt) 1,8%
- Isostéaryl diglycéryle succinate 0,6%
- Isododécane 15,2%

Phase A2:

- Cyclopentasiloxane 5,0%
- Oxydes de fer enrobés de stéaroyl glutamate d'aluminium 3,2%
- Dioxyde de titane enrobé de stéaroyl glutamate d'aluminium 6,8%

Phase A3:

- Polyuréthanne* 15% (soit 2,1% MS)
- Ethanol 2,0%

Phase A4:

- Poudre de Nylon 12 8,0%

Phase B:

- conservateur q.s.
- Sulfate de magnésium 0,7%
- Eau qsp 100%

* copolymère polyuréthane-polyurée-poly(éthylène-butylène) portant une fonction ionisable préparé selon l'exemple 5 de EP1797868, en solution à 14% dans l'isododécane.

On pèse les constituants de la phase A1 dans un bécher, sous agitation en maintenant à température ambiante. La phase A2 est préparée séparément en broyant les pigments à la tricylindre puis elle est ajoutée sous agitation au mélange précédent. La phase A3 est préparée séparément en mélangeant sous agitation le polymère avec l'éthanol. Elle est ensuite introduite dans le mélange précédent sous agitation en maintenant à température ambiante. Puis on ajoute la phase A4 en maintenant l'agitation. Pour préparer la phase B aqueuse, on porte l'eau à l'ébullition puis on ajoute les autres constituants de la phase B. L'émulsion se fait à température ambiante. Les deux phases doivent avoir une température voisine de l'ambiante. On verse la phase aqueuse B lentement dans la phase grasse en augmentant au fur et à mesure la vitesse d'agitation du Moritz jusqu'à 4000 tours/min. Après addition, on laisse agiter encore pendant 10 minutes à température ambiante. On obtient un fond de teint comparatif liquide.

2/ Mesure du non transfert

On mesure l'indice de transfert en présence de sébum et de sueur pour la composition de l'exemple 6 et la composition comparative ci-dessus, selon le protocole de mesure décrit précédemment. On obtient les résultats suivants :

| | Exemple 6 (invention) | Exemple comparatif |
|---|---|---|
| Indice de transfert - Sueur | 0 | 0,5 |

(suite)

| | Exemple 6 (invention) | Exemple comparatif |
|---|---|---|
| Indice de transfert - Sébum | 1,5 | 3,5 |

On constate que la composition de l'invention a un indice de transfert, en présence de sébum ou de sueur, meilleur que celui de la composition de l'art antérieur.

**Exemple 8 : Stick de rouge à lèvres**

On prépare un rouge à lèvres sous forme de stick (bâton) comprenant (% en poids):

| | | % en poids |
|---|---|---|
| A | Cire de polyéthylène | 11,0 |
| | Polyisobutene hydrogéné | 5,7 |
| | Sucrose Acetate Isobutyrate | 5,0 |
| | C30-50 Alcohols | 2,0 |
| | Polyhydroxystearic Acid | 0,2 |
| | Pigments | 3,6 |
| B | Mica | 1,5 |
| | Dioxyde de titane | 3,2 |
| C | Polymère de l'exemple 1 en solution à 15% dans l'isododécane | qsp 100% (soit 10% MS) |

On pèse dans un poêlon double paroi la phase A, puis on chauffe à 95°C sous agitation. Lorsque le mélange est fondu et homogène, on incorpore la phase B, et on laisse agiter pendant 5 minutes. On incorpore la phase C puis on couvre rapidement. On laisse agiter pendant 10 minutes puis on coule dans un moule de type stylo 8 mm. On obtient après refroidissement un stick de rouge à lèvres.

La composition est testée in vitro selon le test de "Push & Pull" consistant à évaluer la résistance de la formule à l'eau et à l'huile.

On colle une plaquette de Blenderme sur une mousse, on porte le tout à 33°C, puis on y applique la composition à tester et on laisse sécher 10 minutes. On y découpe une éprouvette et on en mesure la couleur (L, a, b) (film) à l'aide d'un Chromamètre CR200. On prépare une éprouvette témoin, sans dépôt de composition, et on en mesure également la couleur (L, a, b) (film nu). On monte l'éprouvette à tester sur une presse dynamométrique, on applique une pression de 300 g/$cm^2$ (ou 760 g), et on tire manuellement la bande de papier sur toute sa longueur avec une vitesse de 1 cm/sec. On mesure ensuite la couleur résiduelle du film (L, a, b) (résiduel). On calcule la tenue de la manière suivante :

$$\text{Tenue (\%)} = 100 \times [\ \Delta E(\text{résiduel/film nu}) / \Delta E(\text{film/film nu})\ ]$$

On obtient le résultat suivant : tenue in vitro : 94,58 $\pm$ 0,88 %

On constate donc que la composition selon l'invention possède de bonnes propriétés de tenue.

**Revendications**

**1.** Procédé de maquillage de la peau et/ou des lèvres, dans lequel on applique sur la peau et/ou les lèvres une composition cosmétique de maquillage comprenant, dans un milieu cosmétiquement acceptable, au moins un polymère supramoléculaire à base de polyalcène susceptible d'être issu de la réaction,

- d'au moins un polymère polyalcène fonctionnalisé par au moins un groupe réactif, ledit polymère polyalcène étant de formule HX-P-X'H dans laquelle :

- XH et X'H sont des groupes réactifs, avec X et X' désignent O ;
- P représente un polyéthylène, un polybutylène, un polybutadiène, un polyisoprène, un poly(1,3-pentadiène), un polyisobutylène, et leurs copolymères ;

avec au moins un groupe de jonction fonctionnalisé par au moins un groupe réactif susceptible de réagir avec le ou les groupes réactifs du polymère polyalcène fonctionnalisé, ledit groupe de jonction étant capable de former au moins 3 liaisons H (hydrogène) et étant de formule :

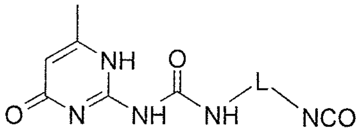

dans laquelle L est un groupe divalent isophorone ou 4,4'-méthylène biscyclohexylène,
le polymère supramoléculaire répondant à la formule :

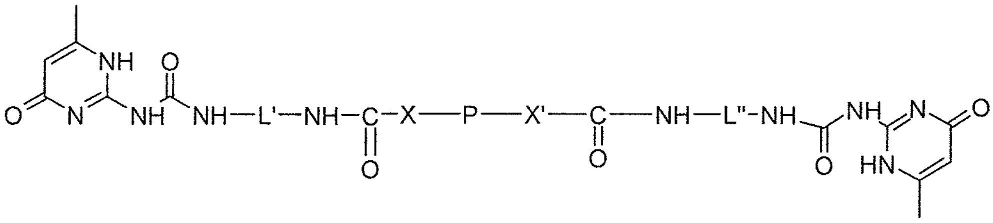

dans laquelle :

- L' et L" sont un groupement -isophorone- ou L' et L" sont un groupement 4,4'-méthylène biscyclohexylène ; et
- X et X' désignent O ; et
- P représente un polyéthylène, un polybutylène, un polybutadiène, un polyisoprène, un poly(1,3-pentadiène), un polyisobutylène, et leurs copolymères.

**2.** Procédé selon la revendication précédente, dans lequel le polymère supramoléculaire à base de polyalcène, seul ou en mélange, est présent dans la composition cosmétique de maquillage à raison de 0,1 à 70% en poids, de préférence de 0,2 à 40% en poids, préférentiellement de 0,5 à 15% en poids, par rapport au poids total de la composition.

**3.** Procédé selon l'une des revendications précédentes, dans lequel la composition se présente sous forme d'une émulsion comprenant (% en poids) :

- de 1 à 80% de phase aqueuse,
- de 0,1 à 10% de tensioactif(s),
- de 1 à 90% de phase grasse,
- de 5 à 20% de matière(s) colorante(s),
- de 0,1 à 20% de charge(s) et
- de 0,1 à 10% de polymère(s) supramoléculaire(s) à base de polyalcène;

ladite composition étant de préférence un fond de teint.

**4.** Procédé selon l'une des revendications 1 à 2, dans lequel la composition est anhydre et comprend (% en poids) :

- de 1 à 20% de matière(s) colorante(s),
- de 1 à 50% de cire(s),
- de 1 à 30% de pâteux,
- de 1 à 60% d'huile(s),
- de 0,1 à 10% de charge(s),

- de 0,1 à 70% de polymère(s) supramoléculaire(s) à base de polyalcène;

ladite composition étant de préférence un rouge à lèvres.

**5.** Composition cosmétique de maquillage de la peau et/ou des lèvres, comprenant, dans un milieu cosmétiquement acceptable, au moins un polymère supramoléculaire à base de polyalcène, et au moins une matière colorante; ledit polymère supramoléculaire à base de polyalcène étant susceptible d'être issu de la réaction:

- d'au moins un polymère polyalcène fonctionnalisé par au moins un groupe réactif, ledit polymère polyalcène étant de formule HX-P-X'H dans laquelle :

  - XH et X'H sont des groupes réactifs, avec X et X' désignent O ;
  - P représente un polyéthylène, un polybutylène, un polybutadiène, un polyisoprène, un poly(1,3-pentadiène), un polyisobutylène, et leurs copoly mères ;

avec au moins un groupe de jonction fonctionnalisé par au moins un groupe réactif susceptible de réagir avec le ou les groupes réactifs du polymère polyalcène fonctionnalisé, ledit groupe de jonction étant capable de former au moins 3 liaisons H (hydrogène) et étant de formule :

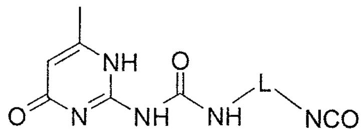

dans laquelle L est un groupe divalent-isophorone- ou 4,4'-méthylène biscyclohexylène ;
le polymère supramoléculaire répondant à la formule :

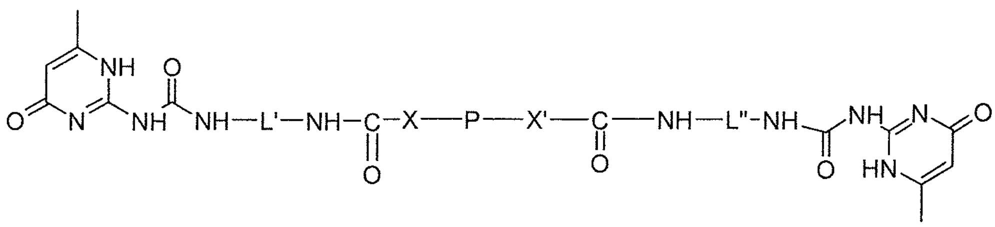

dans laquelle :

  - L' et L" sont un groupement -isophorone- ou L' et L" sont un groupement 4,4'-méthylène biscyclohexylène ; et
  - X et X' désignent O; et
  - P représente un polyéthylène, un polybutylène, un polybutadiène, un polyisoprène, un poly(1,3-pentadiène), un polyisobutylène, et leurs copolymères.

**6.** Composition selon la revendication précédente, dans laquelle le polymère supramoléculaire à base de polyalcène, seul ou en mélange, est présent à raison de 0,1 à 70% en poids, de préférence de 0,2 à 40% en poids, préférentiellement de 0,5 à 15% en poids, par rapport au poids total de la composition.

**7.** Composition selon l'une des revendications 5 ou 6, comprenant au moins une matière colorante présente en une quantité comprise entre 0,001 et 50% en poids, notamment 0,01 et 30% en poids, voire 0,025 et 20% en poids, du poids total de la composition.

**8.** Composition selon l'une des revendications 5 à 7, dans laquelle le milieu cosmétiquement acceptable comprend au moins un ingrédient choisi parmi les charges, les huiles, les cires, les pâteux, de l'eau, les tensioactifs, des agents réducteurs, des adoucissants, des agents anti-mousse, des agents hydratants, des filtres UV, des céramides; des actifs cosmétiques; des peptisants, des parfums, des protéines, des vitamines, des propulseurs, des polymères filmogènes ou non, hydrophiles ou lipophiles; des gélifiants lipophiles ou hydrophiles.

**9.** Composition selon l'une des revendications 5 à 8, se présentant sous forme de produit pour le teint tel qu'un fond de teint, un fard à joues ou à paupières; un produit pour les lèvres tel qu'un rouge à lèvres ou un soin des lèvres; un produit anticernes; un blush, un eye-liner; un crayon à lèvres ou à yeux; un produit de maquillage du corps; un gloss (brillant à lèvres).

**10.** Composition selon l'une des revendications 5 à 9, se présentant sous forme d'un fond de teint et dans laquelle le polymère supramoléculaire à base de polyalcène, seul ou en mélange, est présent à raison de 0,1 à 10% en poids par rapport au poids total de la composition.

**11.** Composition selon la revendication 10, se présentant sous forme d'une émulsion comprenant (% en poids) :

- de 1 à 80% de phase aqueuse,
- de 0,1 à 10% de tensioactif(s),
- de 1 à 90% de phase grasse,
- de 5 à 20% de matière(s) colorante(s),
- de 0,1 à 20% de charge(s) et
- de 0,1 à 10% de polymère(s) supramoléculaire(s) à base de polyalcène.

**12.** Composition selon l'une des revendications 5 à 11, se présentant sous forme d'un rouge à lèvres et dans laquelle le polymère supramoléculaire à base de polyalcène, seul ou en mélange, est présent à raison de 0,1 à 70% en poids, par rapport au poids total de la composition.

**13.** Composition selon la revendication 12, se présentant sous forme d'une composition anhydre comprenant (% en poids) :

- de 1 à 20% de matière(s) colorante(s),
- de 1 à 50% de cire(s),
- de 1 à 30% de pâteux,
- de 1 à 60% d'huile(s),
- de 0,1 à 10% de charge(s),
- de 0,1 à 70% de polymère(s) supramoléculaire(s) à base de polyalcène.

**Patentansprüche**

**1.** Verfahren zum Schminken der Haut und/oder der Lippen, bei dem man auf die Haut und/oder die Lippen eine kosmetische Scbzainkzusammensetzung aufbring, die in einem kosmetisch unbedenklichen Medium mindestens ein supramolekulares Polymer auf Polyalken-Basis umfasst, das aus der Umsetzung

- von mindestens einem mit mindestens einer reaktiven Gruppe funktionalisierten Polyalken-Polymer, wobei das Polyalken-Polymer die Formel HX-P-X'H aufweiset, in der:

  - XH und X'H reaktive Gruppen sind, wobei X und X' O bedeuten;
  - P für ein Polyethylen, ein Polybutylen, ein Polybutadien, ein Polyisopren, ein Pol (1, 3-pentadien), ein Polyisobutylen und Copolymere davon steht;

mit mindestens einer Verknüpfungsgruppe, die mit mindestens einer reaktiven Gruppe funktionalisiert ist, die mit der reaktiven Gruppe bzw. den reaktiven Gruppen des funktionalisierten Polyalken-Polymers reagieren kann, wobei die Verknüpfungsgruppe zur Bindung von mindestens 3 H-Brückenbiri.dungen (Wasserstöffbrückenbindungen) befähigt ist und die Formel:

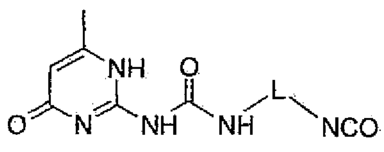

aufweist, in der L eine zweiwertige Isophoron- oder 4,4'-Methylenbiscyclohexylengruppe ist, erhalten werden

kann,
wobei das supramolekulare Polymer der Formel:

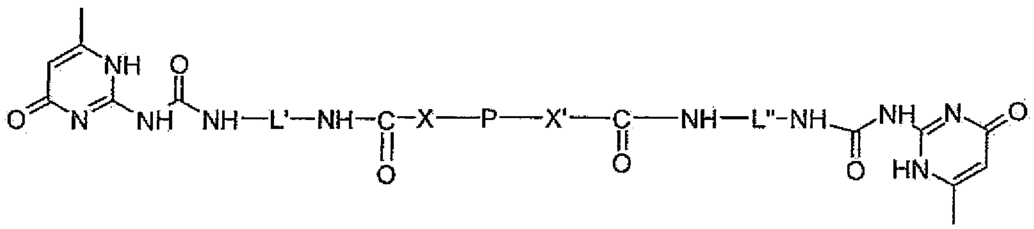

entspricht, in der:

- L' und L'' eine -Isophoron-Gruppierung sind oder L' und L'' eine 4,4'-Methylenbiscyclohexylengruppe sind und
- X und X' 0 bedeuten und
- P für ein Polyethylen, ein Polybutylen, ein Polybutadien, ein Polyisopren, ein Poly(1,3-pentadien), ein Polyisobutylen und Copolymere davon steht.

**2.** Verfahren nach dem vorhergehenden Anspruch, bei dem das supramolekulare Polymer auf Polyalken-Basis allein oder als Gemisch in der kosmetischen Schminkzusammensetzung in einem Anteil von 0,1 bis 70 Gew.-%, vorzugsweise 0,2 bis 40 Ges.-%, bevorzugt 0,5 bis 15 Ges.-%, bezogen auf das Gesamtgewicht der Zusammensetzung, vorliegt.

**3.** Verfahren nach einem der vorhergehenden Ansprüche, bei dem die Zusammensetzung in Form einer Emulsion vorliegt, die Folgendes umfasst (Gew.-%) :

- 1 bis 80% wässrige Phase,
- 0,1 bis 10 % Tensid(e),
- 1 bis 90 % Fettphase,
- 5 bis 20 % Farbstoff(e),
- 0,1 bis 20 % Füllstoff(e) und
- 0,1 bis 10 % supramolekulare(s) Polymer(e) auf Polyalken-Basis;

wobei es sich bei der Zusammensetzung vorzugsweise um eine Make-up-Grundierung handelt.

**4.** Verfahren nach einem der Ansprüche 1 bis 2, bei dem die Zusammensetzung wasserfrei ist und die Folgendes umfasst (Gew.-%):

- 1 bis 20 % Farbstoff(e),
- 1 bis 50 % Wachs(e),
- 1 bis 30 % pastöse Substanzen,
- 1 bis 60 % Öl(e),
- 0,1 bis 10% Füllstoff(e),
- 0,1 bis 70 % supramolekulare(s) Polymer(e) auf Polyalken-Basis;

wobei es sich bei der Zusammensetzung vorzugsweise um einen Lippenstift handelt.

**5.** Kosmetische Zusammensetzung zum Schminken der Haut und/oder der Lippen, die in einem kosmetisch unbedenklichen Medium mindestens ein supramolekulares Polymer auf Polyalken-Basis und mindestens einen Farbstoff umfasst, wobei das supramolekulare Polymer auf Polyalken-Basis aus der Umsetzung

- von mindestens einem mit mindestens einer reaktiven Gruppe funktionalisierten Polyalken-Polymer, wobei das Polyalken-Polymer die Formel HX-P-X'H aufweist, in der:

  - XH und X'H reaktive Gruppen sind, wobei X und X' 0 bedeuten;
  - P für ein Polyethylen, ein Polybutylen, ein Polybutadien, ein Polyisopren, ein Poly (1,3-pentadien), ein

Polyisobutylen und Copolymere davon steht;

mit mindestens einer Verknüpfungsgruppe, die mit mindestens einer reaktiven Gruppe funktionalisiert ist, die mit der reaktiven Gruppe bzw. den reaktiven Gruppen des funktionalisierten Polyalken-Polymers reagieren kann, wobei die Verknüpfungsgruppe zur Bildung von mindestens 3 H-Brückenbindungen (Wasserstoffbrückenbindungen) befähigt ist und die Formel:

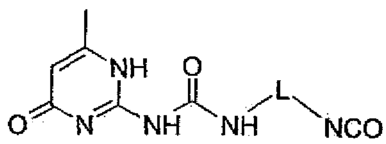

aufweiset, in der L eine zweiwertige -Isophoron- oder 4,4'-Methylenbiscyclohexylengruppe ist, erhalten werden kann,
wobei das supramolekulare Polymer der Formel:

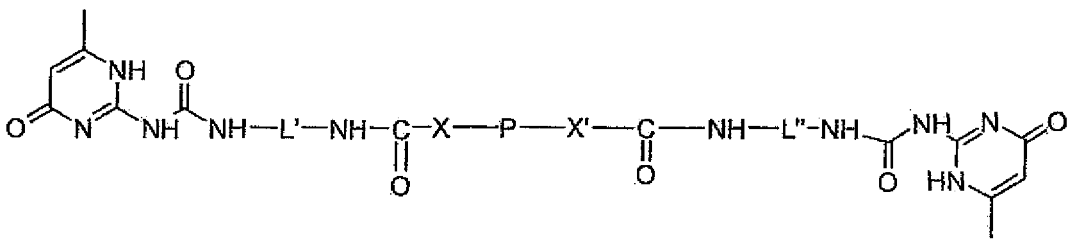

entspricht, in der:

- L' und L'' eine -Isophoron-Gruppierung sind oder L' und L" eine 4,4'-Methylenbiscyclohexylengruppe sind und
- X und X' O bedeuten und
- P für ein Polyethylen, ein Polybutylen, ein Polybutadien, ein Polyisopren, ein Poly(1,3-pentadien), ein Polyisobutylen und Copolymere davon steht.

**6.** Zusammensetzung nach dem vorhergehenden Anspruch, bei dem das supramolekulare Polymer auf Polyalken-Basis allein oder als Gemisch in einem Anteil von 0,1 bis 70 Gew.-%, vorzugsweise 0,2 bis 40 Gew.-%, bevorzugt 0,5 bis 15 Ges.-%, bezogen auf das Gesamtgewicht der Zusammensetzung, vorliegt.

**7.** Zusammensetzung nach Anspruch 5 oder 6, umfassend mindestens einen Farbstoff, der in einer Menge zwischen 0,001 und 50 Gew.-%, insbesondere 0,01 und 30 Gew.-% oder sogar 0,025 und 20 Gew.-%, des Gesamtgewichts der Zusammensetzung vorliegt.

**8.** Zusammensetzung nach einem der Ansprüche 5 bis 7, wobei das kosmetisch unbedenkliche Medium mindestens einen Bestandteil umfasst, der aus Füllstoffen, Ölen, Wachsen, pastösen Substanzen, Wasser, Tensiden, Reduktionsmitteln, Weichmachern, Antischaummitteln, Feuchtigkeitsmitteln, UV-Filtern, Ceramiden; kosmetischen Wirkstoffen; Peptisierungsmitteln, Duftstoffen, Proteinen, Vitaminen, Treibmittel, hydrophilen oder lipophilen filmbildenden oder nicht filmbildenden Polymeren und hydrophilen oder lipophilen Gelbildnern ausgewählt ist.

**9.** Zusammensetzung nach einem der Ansprüche 5 bis 8, die in Form eines Produkts für den Teint wie einer Make-up-Gruxadierung, eines Wangenrouges oder eines Lidschattens; eines Produkts für die Lippen wie eines Lipperastifts oder eines Lippenpflegeprodukts; eines Produkts gegen Falten; eines Rouges, eines Eyeliners; eines Lippenkonturenstifts oder eines Lidschattenstifts; eines Körperschminkproduktes oder eines Lipgloss vorliegt.

**10.** Zusammensetzung nach einem der Ansprüche 5 bis 9, die in Form einer Make-up-Grundierung vorliegt und in der das supramolekulare Polymer auf Polyalken-Basis allein oder als Gemisch in einem Anteil von 0,1 bis 10 Ges.-%, bezogen auf das Gesamtgewicht der Zusammensetzung, vorliegt.

**11.** Zusammensetzung nach Anspruch 10, die in Form einer Emulsion vorliegt, die Folgendes umfasst (Gew.-%):

- 1 bis 80 % wässrige Phase,
- 0,1 bis 10 % Tensid(e),
- 1 bis 90 % Fettphase,
- 5 bis 20 % Farbstoff(e),
- 0,1 bis 20 % Füllstoff(e) und
- 0,1 bis 10 % supramolekulare(s) Polymer(e) auf Polyalken-Basis.

**12.** Zusammensetzung nach einem der Ansprüche 5 bis 11, die in Form eines Lippenstifts vorliegt und in der das supramolekulare Polymer auf Polyalken-Basis allein oder als Gemisch in einem Anteil von 0,1 bis 70 Ges.-%, bezogen auf das Gesamtgewicht der Zusammensetzung, vorliegt.

**13.** Zusammensetzung nach Anspruch 12, die in Form einer wasserfreien Zusammensetzung vorliegt, die Folgendes umfasst (Gew.-%) :

- 1 bis 20% Farbstoff(e),
- 1 bis 50 % Wachs(e),
- 1 bis 30 % pastöse Substanzen,
- 1 bis 60 % Öl(e),
- 0,1 bis 10 % Füllstoff(e),
- 0,1 bis 70 % supramolekulare(s) Polymer(e) auf Polyalken-Basis.

## Claims

**1.** Method for making up the skin and/or lips, in which a cosmetic makeup composition comprising, in a cosmetically acceptable medium, at least one polyalkene-based supramolecular polymer is applied to the skin and/or lips, which polyalkene-based supramolecular polymer is capable of resulting from the reaction,

- of at least one polyalkene polymer functionalized by at least one reactive group, the said polyalkene polymer being of formula HX-P-X'H, in which:

  - XH and X'H are reactive groups with X and X' denoting O ;
  - P represents a polyethylene, a polybutylene, a polybutadiene, a polyisoprene, a poly(1,3-pentadiene), a polyisobutylene and their copolymers;

with at least one joining group functionalized by at least one reactive group capable of reacting with the reactive group or groups of the functionalized polyalkene polymer, the said joining group being capable of forming at least 3 H (hydrogen) bonds and being of formula:

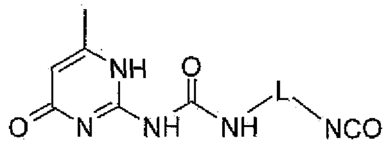

in which L is a divalent isophorone or 4,4'-methylenebiscyclohexylene group,
the supramolecular polymer corresponding to the formula:

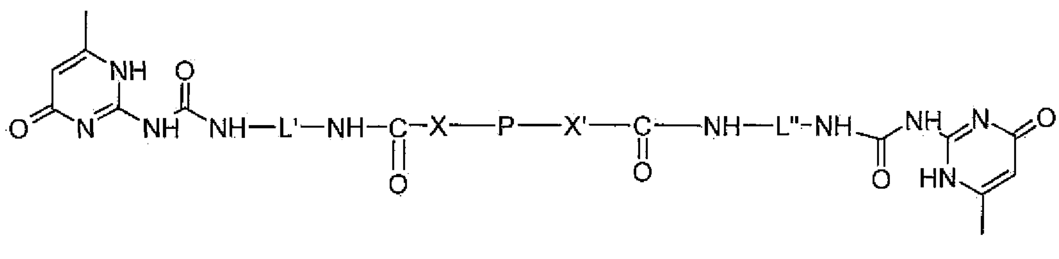

in which:

- L' and L" are an isophorone group or L' and L" are a 4,4'-methylenebiscyclohexylene group; and
- X and X' denote O ; and
- P represents a polyethylene, a polybutylene, a polybutadiene, a polyisoprene, a poly (1,3-pentadiene), a polyisobutylene and their copolymers.

**2.** Method according to the preceding claim, in which the polyalkene-based supramolecular polymer, alone or as a mixture, is present in the cosmetic makeup composition in a proportion of 0.1 to 70% by weight, preferably of 0.2 to 40% by weight, preferentially of 0.5 to 15% by weight, with respect to the total weight of the composition.

**3.** Method according to either of the preceding claims, in which the composition is provided in the form of an emulsion comprising (% by weight) :

- from 1 to 80% of aqueous phase,
- from 0.1 to 10% of surfactant(s),
- from 1 to 90% of fatty phase,
- from 5 to 20% of colouring material(s),
- from 0.1 to 20% of filler(s) and
- from 0.1 to 10% of polyalkene-based supramolecular polymer(s);

the said composition preferably being a foundation.

**4.** Method according to either of Claims 1 and 2, in which the composition is anhydrous and comprises (% by weight) :

- from 1 to 20% of colouring material(s),
- from 1 to 50% of wax(es),
- from 1 to 30% of pasty substance(s),
- from 1 to 60% of oil(s),
- from 0.1 to 10% of filler(s),
- from 0.1 to 70% of polyalkene-based supramolecular polymer(s);

the said composition preferably being a lipstick.

**5.** Cosmetic composition for making up the skin and/or lips, comprising, in a cosmetically acceptable medium, at least one polyalkene-based supramolecular polymer and at least one colouring material; the said polyalkene-based supramolecular polymer being capable of resulting from the reaction:

- of at least one polyalkene polymer functaonalized by at least one reactive group, the said polyalkene polymer being of formula HX-P-X'H, in which:
- XH and X'H are reactive groups with X and X' denoting O ;
- P represents a polyethylene, a polybutylene, a polybutadiene, a polyisoprene, a poly(1,3-pentadiene), a polyisobutylene and their copolymers;

with at least one joining group functionalized by at least one reactive group capable of reacting with the reactive group or groups of the functionalized polyalkene polymer, the said joining group being capable of forming at least 3 H (hydrogen) bonds and being of formula:

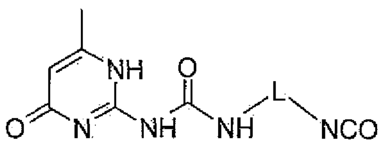

in which L is a divalent isophorone or 4,4'-methylenebiscyclohexylene group;
the supramolecular polymer corresponding to the formula:

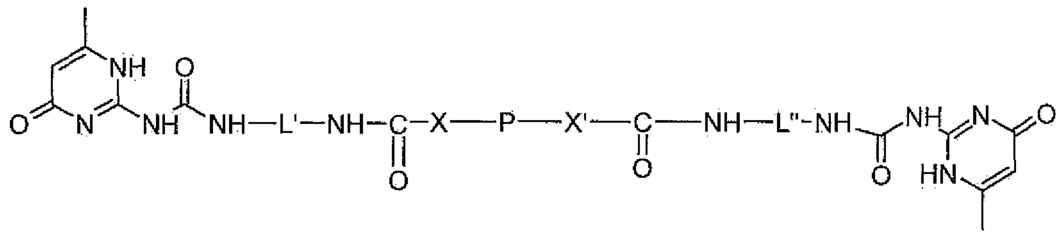

in which:

- L' and L" are an isophorone group or L' and L" are a 4,4'-methylenebiscyclohexylene group; and
- X and X' denote O ; and
- P represents a polyethylene, a polybutylene, a polybutadiene, a polyisoprene, a poly(1,3-pentadiene), a polyisobutylene and their copolymers.

**6.** Composition according to the preceding claim, in which the polyalkene-based supramolecular polymer, alone or as a mixture, is present in a proportion of 0.1 to 70% by weight, preferably of 0.2 to 40% by weight. preferentially of 0.5 to 15% by weight, with respect to the total weight of the composition.

**7.** Composition according to either of Claims 5 and 6, comprising at least one colouring material present in an amount of between 0.001 and 50% by weight, in particular 0.01 and 30% by weight, indeed even 0.025 and 20% by weight, of the total weight of the composition.

**8.** Composition according to one of Claims 5 to 7, in which the cosmetically acceptable medium comprises at least one ingredient chosen from fillers, oils, waxes, pasty substances, water, surfactants, reducing agents, softeners, antifoaming agents, moisturizing agents, UV screening agents, ceramides, cosmetic active principles, peptizing agents, fragrances, proteins, vitamins, propellents, hydrophilic or lipophilic polymers which are or are not able to form a film, or lipophilic or hydrophilic gelling agents.

**9.** Composition according to one of Claims 5 to 8, which is provided in the form of a product for the complexion, such as a foundation, a face powder or an eyeshadow; a product for the lips, such as a lipstick or a lipcare product; a concealer; a blusher; an eyeliner; a lip or eye pencil; a product for making up the body; or a gloss (lip gross).

**10.** Composition according to one of Claims 5 to 9, which is provided in the form of a foundation and in which the polyalkene-based supramolecular polymer, alone or as a mixture, is present in a proportion of 0.1 to 10% by weight, with respect to the total weight of the composition.

**11.** Composition according to Claim 10, which is provided in the form of an emulsion comprising (% by weight) :

- from 1 to 80% of aqueous phase,
- from 0.1 to 10% of surfactant(s),
- from 1 to 90% of fatty phase,
- from 5 to 20% of colouring material(s),
- from 0.1 to 20% of filler(s) and
- from 0.1 to 10% of polyalkene-based supramolecular polymer(s).

**12.** Composition according to one of Claims 5 to 11, which is provided in the form of a lipstick and in which the polyalkene-based supramolecular polymer, alone or as a mixture, is present in a proportion of 0.1 to 70% by weight, with respect to the total weight of the composition.

**13.** Composition according to Claim 12, which is provided in the form of an anhydrous composition comprising (% by weight) :

- from 1 to 20% of colouring material(s),
- from 1 to 50% of wax(es),
- from 1 to 30% of pasty substance(s),
- from 1 to 60% of oil(s),

- from 0.1 to 10% of filler(s),
- from 0,1 to 70% of polyalkene-based supramolecular polymer(s).

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0209837 A
- WO 03032929 A
- WO P1797868 A
- FR 2679771
- EP 1184426 A
- US 4578266 A
- US 6491927 B
- EP 1797868 A


### Littérature non-brevet citée dans la description


- *Cosmetics and Toiletries,* Février 1990, vol. 105, 53-64